# EUROPEAN PATENT APPLICATION

(11) **EP 3 175 771 A1**
(43) Date of publication of application: **07.06.2017**
(21) Application number: 16792413.3
(22) Date of filing: 01.03.2016
(51) Int. Cl.: A61B 1/00, G02B 23/24, G02B 23/26

(54) **ENDOSCOPE**

(30) Priority: 13.05.2015 JP 2015098450
(71) Applicant: Olympus Corporation, Tokyo 192-8507 (JP)
(72) Inventor: TATEBAYASHI, Takaaki, Tokyo 192-8507 (JP)
(74) Representative: Schicker, Silvia
(86) International application number: PCT/JP2016/056276
(87) International publication number: WO 2016/181688

(57) **Abstract**

An endoscope 2 is configured such that, at a distal end portion 15 of an insertion portion 11, an objective lens 118 is formed at a position protruded most in a distal end direction, a head top surface of a head top portion 34b of an air/water feeding nozzle 34 is formed at a position protruded in the distal end direction, and then two (first and second) illumination portions 29a, 29b are formed at a position protruded in the distal end direction.

## Description

### Technical Field

The present invention relates to an endoscope configured to be switchable between observation in a contact state with respect to an observation object and observation in a non-contact state with respect to the observation object.

### Background Art

In recent years, endoscopes capable of switching between an observation in a contact state with respect to an observation object and observation in a non-contact state with respect to the observation object by switching a focus of an objective optical system has been put into practical use. For example, Patent Document 1 discloses an endoscope including, on a plane of a protruding step portion at a distal end of an insertion portion to be inserted into a subject, an observation window of an observation portion configured to observe the subject in a contact state or a proximate state, and an illumination window of an illumination system that applies illumination light toward an observation field of view of the observation portion, and configured to be switchable between the observation in the contact state and the observation in the non-contact state.

In the technique of the endoscope disclosed in Japanese Patent Application Laid-Open Publication No. 2008-86664, when an endoscopic observation in a body cavity and the like is performed, for example, since the illumination window of the illumination system and the observation window of the observation portion are disposed on the same plane, if a long-time observation is performed with the observation window of the observation portion being brought into contact with the site to be observed in the body cavity and the like, also the illumination window contacts the site to be observed and the living mucosa of the site to be observed is likely to be affected by the heat of the illumination light.

The present invention has been achieved in view of the above-described circumstance, and an object of the present invention is to provide an endoscope with improved observation performance, which is capable of sufficiently performing observation without having any influence of heat on a site to be observed even if a long-time observation is performed with an observation window of an observation portion being brought into contact with the site to be observed in the body cavity and the like.

### Disclosure of Invention

### Means for Solving the Problem

An endoscope according to one aspect of the present invention includes: an insertion portion formed so as to extend in a longitudinal direction and configured to be inserted into a subject; an observation window arranged at a distal end of the insertion portion, and configured to allow at least observation in a state of contacting an observation object; and an illumination window configured to apply illumination light toward an observation field of view of the observation portion, wherein the illumination window is arranged on a proximal end side in the longitudinal direction with respect to the observation window, and a distal end of a protruding portion is arranged between the observation window and the illumination window in the longitudinal direction.

### Brief Description of the Drawings

FIG. 1 is a schematic configuration diagram of a whole endoscope system according to a first embodiment of the present invention.
FIG. 2 is a front view of an endoscope distal end surface according to the first embodiment of the present invention.
FIG. 3 is a cross-sectional view of a main part taken along III-III line in FIG. 2.
FIG. 4 is a cross-sectional view of the main part taken along IV-IV line in FIG. 2.
FIG. 5 is a cross-sectional view of the main part taken along V-V line in FIG. 3.

### Best Mode for Carrying Out the Invention

Hereinafter, an embodiment of the present invention will be described with reference to drawings.

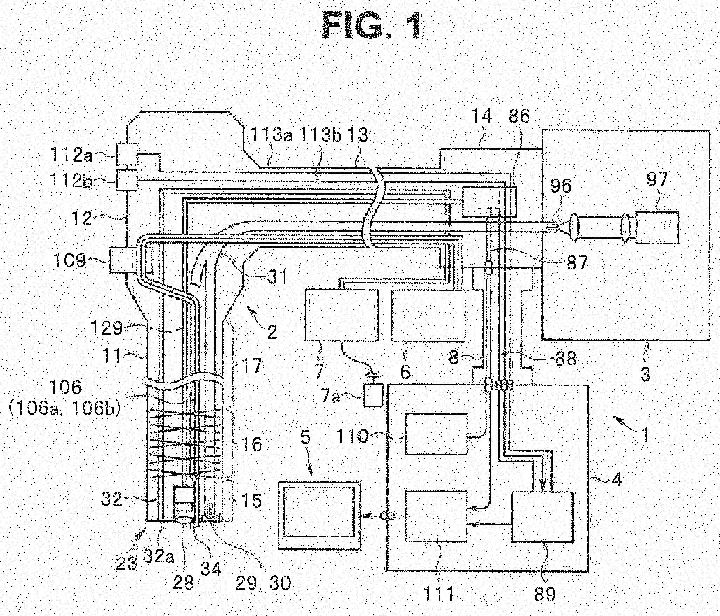

In FIG. 1, the reference sign 1 represents an endoscope system. The endoscope system 1 includes an endoscope 2, a light source apparatus 3 of an illumination system that supplies illumination light to the endoscope 2, a processor 4 as a signal processing apparatus that performs signal processing on the endoscope 2, a monitor 5 connected to the processor 4, and an air/water feeding apparatus 6 that feeds air and water, a forward water feeding apparatus 7 that feeds water in front of the distal end surface of the endoscope 2.

The endoscope 2 includes an elongated insertion portion 11 to be inserted into a body cavity as a subject, for example, an operation portion 12 linked to a proximal end of the insertion portion 11, and a universal cable 13 extended from a side portion of the operation portion 12. A connector 14 provided at an end portion of the universal cable 13 is detachably connected to the light source apparatus 3. In addition, the connector 14 is connected to the processor 4 through a scope cable 8.

The insertion portion 11 of the endoscope 2 is formed in an elongated shape extending along the longitudinal direction, and includes a rigid distal end portion 15 formed at the distal end of the insertion portion 11, a bending portion 16 formed at the proximal end of the distal end portion 15, and a flexible tube portion 17 having flexibility and formed from the proximal end of the bending portion 16 to the operation portion 12.

The bending portion 16 of the endoscope 2 includes a plurality of annular bending pieces, not shown, which are rotatably provided in a linked manner along the axial direction of the insertion portion 11. Each of the bending pieces includes, on the inner circumferential surface thereof, four pipe-shaped wire receivers that are fixed by welding or the like. The four wire receivers are fixed on the inner circumferential surface of the one bending piece at the positions shifted from each other by substantially 90 degrees around the insertion axis.

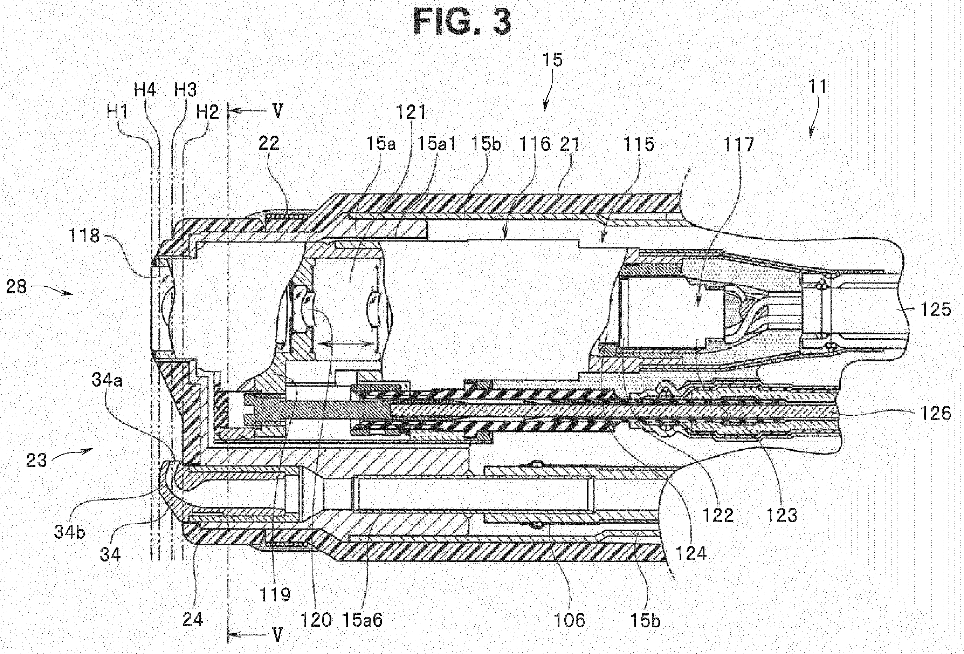

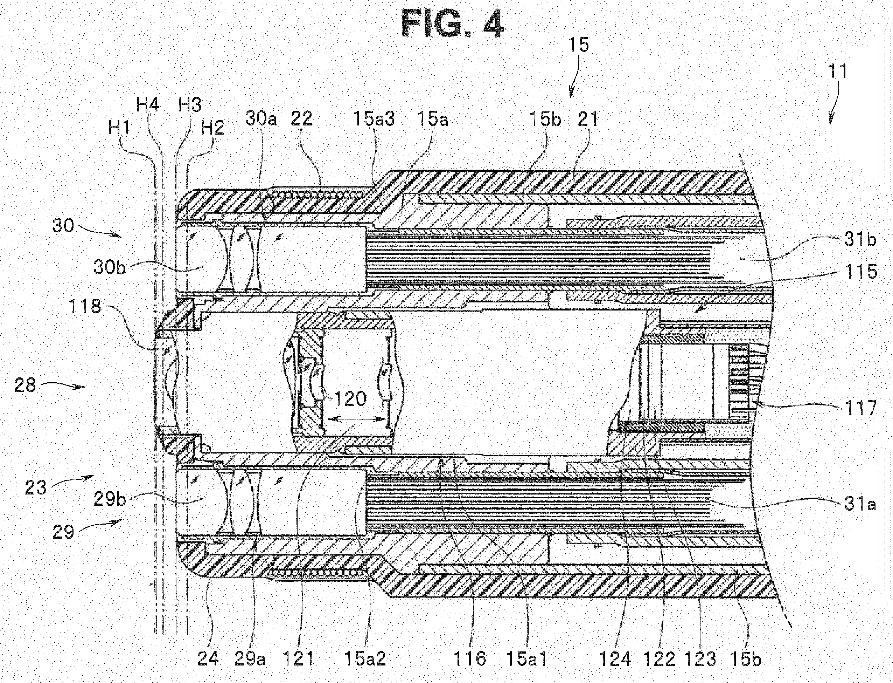

In addition, a bending braid, not shown, formed by weaving thin wires into a cylindrical shape, is put on the plurality of bending pieces so as to cover the outer circumference of the bending pieces. Furthermore, the outside of the bending braid is covered with an outer cover 21 so as to maintain watertightness (see FIGS. 3 and 4).

The insertion portion 11, which is constituted of the distal end portion 15, the bending portion 16, and the flexible tube portion 17, is covered with the outer cover 21 so as to be one unified body over the whole length. The distal end outer circumferential part of the outer cover 21 is secured to the distal end portion 15 with a thread-wound adhering portion 22 which is formed by winding a thread and then applying an adhesive, for example, as shown in FIGS. 3 and 4.

In addition, four bending operation wires, not shown, for bending and operating the bending portion 16 are inserted through the insertion portion 11. The distal end parts of these four bending operation wires are held and fixed by four fixing portions of a fixing ring provided in the distal end portion 15 such that the four bending operation wires are shifted from each other by substantially 90 degrees around the insertion axis. Furthermore, the four bending operation wires are inserted respectively through the wire receivers on the inner circumferential surface of each of the bending pieces. The four bending operation wires pass through the interiors of the bending portion 16 and the flexible tube portion 17 to be extended toward the operation portion 12 located on the proximal end side. In addition, the fixing ring is inserted and fitted to the inner circumferential side of a reinforcing ring 15b, to be described later, of the distal end portion 15.

Note that the proximal end portions of the bending operation wires are linked to a bending operation mechanism, not shown, provided in the operation portion 12. The operation portion 12 is provided with an operation knob, not shown, for driving the bending operation mechanism by pulling the bending operation wires.

By operating the operation knob, the four bending operation wires are alternately pulled or relaxed, to thereby cause the bending portion 16 to be bent in four directions. The four directions correspond to the four directions, i.e., up, down, left and right directions of the endoscopic image to be displayed on the monitor 5.

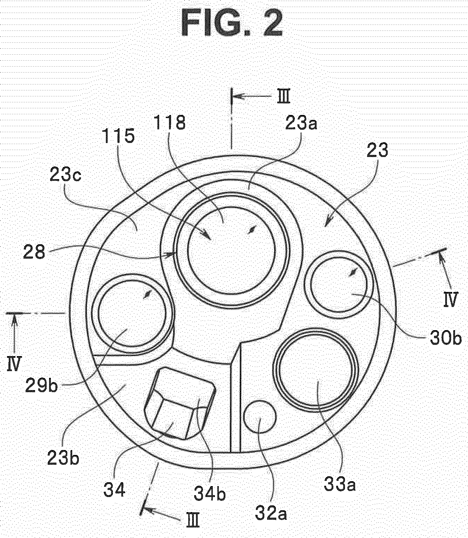

FIG. 2 is a front view of the end surface (distal end surface) 23 of the distal end portion 15 of the insertion portion 11 of the endoscope 2, and the distal end surface 23 of the distal end portion 15 is a surface in the direction substantially orthogonal to the longitudinal direction of the insertion portion 11.

The distal end surface 23 includes a first distal end surface 23a, a second distal end surface 23b, and a third distal end surface 23c, which are formed so as to be adjacent to one another. The adjacent parts of the distal end surfaces 23a to 23c are linked by inclined planes.

The first distal end surface 23a includes an objective lens 118 as an observation window of an observation optical system 115 of an observation portion 28 to be described later, which is switchable between observation in the contact state with respect to the observation object and observation in the non-contact state with respect to the observation object.

In addition, the second distal end surface 23b is provided with an air/water feeding nozzle 34 including a spouting port 34a which is open toward the objective lens 118, and configured to spout fluid from the proximal end side toward the distal end portion of the insertion portion 11 of the endoscope 2.

Furthermore, on the third distal end surface 23c, a first illumination window 29b of a first illumination portion 29 and a second illumination window 30b of a second illumination portion 30 that apply illumination light toward the observation field of view of the observation portion are formed on both sides of the objective lens 118.

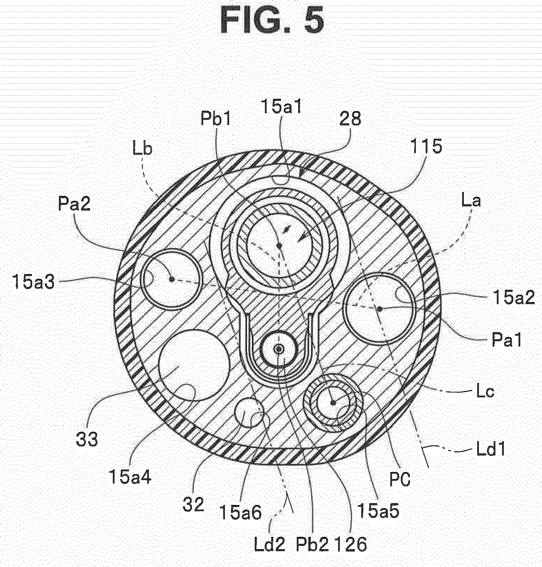

In addition, in the present embodiment, the distal end surface 23 of the distal end portion 15 is provided with a distal end opening portion 33a of a treatment instrument insertion channel (also referred to as forceps channel) 33 and a conduit opening portion 31a of a forward water feeding conduit (forward water feeding channel) 31 for supplying fluid such as cleaning water (see FIG. 5).

The distal end surfaces 23a to 23c are formed at different positions with respect to the longitudinal direction of the insertion portion 11. The following describes the positional relationship among the first distal end surface 23 a, the second distal end surface 23b, and the third distal end surface 23c.

The two-dot-chain lines in FIGS. 3 and 4 show the positions of the distal end surfaces 23a to 23c in the direction substantially orthogonal to the longitudinal direction of the insertion portion 11. The position of the first distal end surface 23a on which the objective lens 118 is provided is designated as H1, the position of the second distal end surface 23b on which the air/water feeding nozzle 34 is provided is designated as H2, and the position of the third distal end surface 23c on which the first illumination window 29b and the second illumination window 30b are provided is designated as H3. Furthermore, in addition to the positions of the distal end surfaces 23a to 23c, the position of a head top surface of a head top portion 34b of the air/water feeding nozzle 34 is designated as H4. As is also clear from FIGS. 3 and 4, the first distal end surface 23a is formed at the position (H1) which protrudes most toward the distal end side along the longitudinal direction, and the head top portion 34b of the air/water feeding nozzle 34 is formed at the position (H4) which is more proximal end side than the position (H1). Furthermore, the third distal end surface 23c is formed at the position (H3) which is more proximal end side than the position (H4). The second distal end surface 23b is formed at the position (H2) which is the most proximal end side.

Therefore, it is easy to bring the objective lens 118 on the first distal end surface 23a protruded in the most distal direction into good contact with the observation object. In addition, even if the objective lens obliquely contacts the observation surface of an observation object (or contacts the observation surface so as to have a gradient), the head top surface (H4) of the head top portion 34b of the air/water feeding nozzle 34 contacts the observation object, to thereby surely prevent the third distal end surface 23c (H3) on which the first illumination window 29b and the second illumination window 30b are provided from contacting the observation object. With such a configuration, even if observation is performed for a long time with the observation window of the observation portion being brought into contact with the site to be observed in the body cavity or the like, the site to be observed is not affected by the heat, which enables the observation to be performed sufficiently and enables the observation performance to be improved.

Though the second distal end surface 23b is provided on the more proximal end side along the longitudinal direction than the third distal end surface 23c in the present embodiment, it is sufficient that the head top surface of the head top portion 34b of the air/water feeding nozzle 34 is located on the more distal end side than the first illumination window 29b and the second illumination window 30b. That is, the position of the second distal end surface 23b in the longitudinal direction and the position of the third distal end surface 23c in the longitudinal direction may coincide with each other, and furthermore, the second distal end surface 23b may be provided on the more distal end side along the longitudinal direction than the third distal end surface 23c.

Furthermore, even if the position of the head top portion 34b of the air/water feeding nozzle 34 in the longitudinal direction is made coincident with the position of the objective lens 118 as the observation window (that is, the position of the first distal end surface 23a), the same working effects as described above can be obtained.

Next, detailed description will be made on each of the portions disposed in the distal end portion of the insertion portion 11 of the endoscope 2 according to the present embodiment.

As shown in FIGS. 3 and 4, a cylindrical member made of rigid metal (distal end rigid member) 15a and the annular-shaped reinforcing ring 15b fitted on the proximal end side outer circumferential portion of the cylindrical member 15a are disposed in the distal end portion 15 of the insertion portion 11. The cylindrical member 15a includes a plurality of holes which are parallel to the axial direction of the insertion portion 11, i.e., six holes, that is, a first through hole 15a1 to a sixth through hole 15a6 (only the first through hole 15a1 and the sixth through hole 15a6 are shown in FIG. 3) in the present embodiment. The proximal end part of the reinforcing ring 15b is linked to the distal-most bending piece.

In addition, the first through hole 15a1 to the third through hole 15a3 are formed at the positions respectively corresponding to the observation portion 28, the first illumination portion 29, and the second illumination portion 30, and the constituent elements of the observation portion 28, the first illumination portion 29 and the second illumination portion 30 are incorporated in the through holes.

Furthermore, the fourth through hole 15a4 to the sixth through hole 15a6 are formed at the positions respectively corresponding to the distal end opening portion 33a of the treatment instrument insertion channel 33, the air/water feeding nozzle 34, an opening portion 30a of the fluid feeding conduit 30, and the constituent elements of the conduit of the treatment instrument insertion channel 33, the air/water feeding nozzle 34, and the forward water feeding conduit 30 are incorporated in these through holes.

In addition, a distal end cover 24 is mounted so as to be fitted on the distal end surface of the cylindrical member 15a and the distal end side outer circumferential portion of the cylindrical member 15a.

The air/water feeding nozzle 34 provided at the distal end of the fifth through hole 15a5 is a tubular member bent in substantially an L-shape. The distal end portion of the air/water feeding nozzle 34 is arranged so as to face the objective lens 118 of the observation optical system 115 of the observation portion 28. Specifically, the spouting port 34a of the distal end opening portion of the air/water feeding nozzle 34 is disposed opposed to the objective lens 118, with the inclined plane which is between the first distal end surface 23a on which the objective lens 118 is provided and the second distal end surface 23b which is adjacent to the first distal end surface 23a and on which the air/water feeding nozzle 34 is provided, as a guide plane for guiding the fluid to the first distal end surface 23a.

Note that the air/water feeding nozzle 34 is connected to an air/water feeding conduit 106 whose distal end sides are joined and formed into one portion, and the proximal end side of the air/water feeding conduit 106 is branched off into an air feeding conduit 106a and a water feeding conduit 106b, as shown in FIG. 1. The air/water feeding conduit 106 is inserted through to the connector 14 via the insertion portion 11, the operation portion 12 of the endoscope 2, and the universal cable 13, and connected to the air/water feeding apparatus 6. Note that the reference sign 109 shown on the operation portion 12 of the endoscope 2 in FIG. 1 represents an air/water feeding switch for feeding air and water.

Next, description will be made on the observation portion 28.

The observation portion 28 is provided with an observation optical system 115 of a type for contact/non-contact observation. The observation optical system 115 of the type for contact/non-contact observation includes a lens unit 116 that is capable of changing the focusing position between the standard position and the proximate position, and an electric parts unit 117 arranged behind the lens unit 116.

The distal-most end part of the lens unit 116 is the objective lens 118. The lens unit 116 includes inside thereof a moving lens barrel 119 and a moving lens 120 supported by the moving lens barrel 119, as a moving optical unit that is advanceable and retractable with respect to the photographing optical axis.

The lens unit 116 includes inside thereof a guide space 121 for holding the moving lens barrel 119 so as to be advanceable and retractable along the photographing optical axis. A lens, not shown, is disposed also behind the guide space 121.

In the observation optical system 115 configured as described above, a distal end portion of an operation wire 126 for advancing and retracting the moving lens barrel 119a along the photographing optical axis is fixed to the moving lens barrel 119.

The operation wire 126 is formed by a liner member extended from the proximal end side of the insertion portion 11 to be linked to the observation portion 28, and configured to switch the focus position of the observation portion 28 by being advanced or retracted in the longitudinal direction of the insertion portion 11.

The operation wire 126 is driven to be advanced and retracted with respect to the photographing optical axis direction by a lever, not shown, provided to the operation portion 12 of the endoscope 2 being operated by the user.

In accordance with the operation for pushing out the operation wire 126 in the distal end direction, the moving lens barrel 119 is moved forward (standard position direction). In contrast, in accordance with the operation for pulling the operation wire 126 in the hand side direction, the moving lens barrel 119 is moved toward the hand side (proximate position direction).

That is, the state where the moving lens barrel 119 is moved to a position other than the rear-most position of the guide space 121 is set as a range (normal observation mode) in which observation is performed in the non-contact state with respect to the observation object. In addition, the state where the moving lens barrel 119 is moved to the rear-most position of the guide space 121 is set as the observation position where observation is performed in the contact state with respect to the observation object (object contact observation mode in which a monitor observation magnification is approximately 200 to 1000 power). According to such a configuration, operation of a lever, not shown, enables switching between the observation position (normal observation mode) in which the observation object is observed with the moving lens barrel 119 not contacting the observation object and the observation position (object contact observation mode) in which the observation object is observed with the moving lens barrel 119 contacting the observation object.

As described above, the electric parts unit 117 is provided continuously with the rear end portion of the lens unit 116. The electric parts unit 117 includes an image pickup device 122 such as a CCD image sensor, or a CMOS image sensor, and a circuit substrate 123. Furthermore, a cover glass 124 is provided on the light-receiving face side of the front surface of the image pickup device 122.

The cover glass 124 is fixed to the rear end of the lens unit 116. The observation optical system 115 of a type for contact/non-contact observation in which the lens unit 116 and the electric parts unit 117 are unified is thus formed.

The circuit substrate 123 includes, at the lateral side of the rear end part thereof, electric parts and a wiring pattern, and the wiring pattern is connected with distal ends of a plurality of signal lines of a signal cable 125 by means of soldering or the like. Furthermore, the outer circumferential portions of the cover glass 124, image pickup device 122, circuit substrate 123, and distal end parts of the signal cable 125 are covered integrally with an insulating sealing resin or the like.

The optical image formed on the image pickup device 122 through the lens unit 116 is photoelectrically converted into an electric image signal by the image pickup device 122, and the image signal is outputted to the circuit substrate 123. Furthermore, the electric signal of the optical image outputted from the circuit substrate 123 is transmitted to the processor 4 as subsequent electrical equipment, through the signal cable 125.

In addition, the signal cable 125 of the image pickup device 122 passes sequentially through the interiors of the insertion portion 11, the operation portion 12, and the universal cable 13 to be extended into the connector 14, as shown in FIG. 1. The connector 14 incorporates a relay substrate 86. The relay substrate 86 is connected with the proximal end portion of the signal cable 125. The signal cable 125 is connected to a signal cable 87 through the relay substrate 86 in the connector 14.

Furthermore, the relay substrate 86 in the connector 14 is connected to a drive circuit 110 configured to drive the image pickup device 122 of the observation optical system 115, a signal processing circuit 111 configured to perform signal processing on the image pickup signal outputted from the image pickup device 122 through the relay substrate 86, a control circuit 89 configured to control the operation state of the signal processing circuit 111, and the like in the processor 4, through the signal cable 87 in the connector 14 and signal lines 88 in the scope cable 8.

Next, description will be made on the first illumination portion 29 and the second illumination portion 30 that configure the illumination system, with reference to FIG. 4.

The first illumination portion 29 and the second illumination portion 30 have substantially the same configuration. In the first illumination portion 29 and the second illumination portion 30, the distal end parts of the first light guide 31a and second light guide 31b that transmit illumination light are disposed respectively at the rear end portions of the first illumination lens group 29a and second illumination lens group 30a. Each of the first light guide 31a and the second light guide 31b includes at the distal end part thereof a cylindrical member, not shown, that covers the distal end part, and is covered with an outer cover that bundles a plurality of fibers and a protective tube made of Gore-tex material, for example.

In the present embodiment, the first light guide 31a and the second light guide 31b are configured by being branched off from the one light guide bundle 31 in the operation portion 12 of the endoscope 2, for example, and inserted through the insertion portion 11 as divided two parts. The distal end portions of the first light guide 31a and second light guide 31b, which are the divided two parts, are arranged opposed to each other in the vicinity of the rear surfaces of the two illumination windows provided on the distal end cover 24, that is, the first illumination lens group 29a including the first illumination window 29b and the second illumination lens group 30a including the second illumination window 30b, and fixed by screws at the rear end portion of the second through hole 15a2 of the cylindrical member 15a and at the rear end portion of the third through hole 15a3 of the cylindrical member 15a, respectively, for example.

As shown in FIG. 1, the light guide bundle 31 passes sequentially through the interiors of the insertion portion 11, the operation portion 12 of the endoscope 2, and the universal cable 13 to be extended into the connector 14. The proximal end portion side of the light guide bundle 31 is connected to an incident end portion 96 of a light guide bundle connector protruded from the connector 14. The incident end portion 96 of the light guide bundle connector is detachably connected to the light source apparatus 3.

The illumination light emitted from an illumination lamp 97 of the light source apparatus 3 is applied to the incident end portion 96 of the light guide bundle connector, then guided through the light guide bundle 31, to be emitted forward of the endoscope 2 through the first illumination lens group 29a and the second illumination lens group 30a.

Note that description has been made by using the example in which the first illumination portion 29 and the second illumination portion 30 apply the illumination light to the field of view of the observation object in the present embodiment. However, three or more illumination portions may be used to apply the illumination light to the field of view of the observation object.

A distal end part of a communication tube that communicates with the treatment instrument insertion channel 33 is inserted from the proximal end portion side of the fourth through hole 15a4 formed in the cylindrical member 15a of the distal end portion 15 and fitted to the fourth through hole 15a4. The proximal end portion of the communication tube is protruded to the rearward of the cylindrical member 15a, and the proximal end part of the communication tube is linked with the distal end portion of the treatment instrument insertion channel 33. The distal end of the treatment instrument insertion channel 33 is communicated with the distal end opening portion 33a of the distal end cover 24 through the communication tube.

The treatment instrument insertion channel 33 is branched off in the vicinity of the proximal end of the insertion portion 11, and one of the branched-off parts is inserted through to the treatment instrument insertion port, not shown, disposed at the operation portion 12 of the endoscope 2. The other of the branched-off parts passes through the interiors of insertion portion 11 and universal cable 13, to communicate with a suction channel, and the proximal end of the other of the branched-off parts is connected to suction means, not shown, through the connector 14.

In addition, a distal end part of a substantially cylindrical-shaped tubular member, not shown, is inserted from the rear end portion side of the sixth through hole 15a6 formed in the cylindrical member 15a of the distal end portion 15 and fitted to the sixth through hole 15a6. The proximal end portion of the tubular member is protruded to the rearward of the cylindrical member 15a, and the proximal end part of the tubular member is linked with the distal end portion of the forward water feeding conduit 30. Note that the distal end portion of the forward water feeding conduit 30 covers the proximal end part of the tubular member and the distal end part of the forward water feeding conduit is connected and fixed by a thread-wound portion.

The forward water feeding conduit 30 passes through the insertion portion 11, the operation portion 12 of the endoscope 2, and the universal cable 13 to the connector 14, and is connected to the forward water feeding apparatus 7. In the operation portion 12 of the endoscope 2, a forward water feeding button, not shown, is disposed at the middle portion of the forward water feeding conduit 30.

When the forward water feeding button is operated, liquid such as sterilized water is sprayed from the opening portion 30a of the distal end cover 24 of the insertion portion 11 toward the insertion direction into the body cavity. This enables to clean body fluid adhered to the site to be examined in the body cavity. Note that, as shown in FIG. 1, a foot switch 7a is connected to a cable extended from the forward water feeding apparatus 7, and the user can spray the liquid such as sterilized water from the opening portion 30a of the distal end cover 24 of the insertion portion 11 toward the insertion direction into the body cavity by operating the foot switch 7a.

According to the configuration of the endoscope 2 as described above, the operation portion 12 of the endoscope 2 includes control switches 112a, 112b connected with the relay substrate 86 in the connector 14 through signal lines 113a, 113b, an air/water feeding button 109, a bending operation knob, not shown, a lever, not shown, for performing focusing position changing operation of the observation optical system 115, the forward water feeding button, not shown, and the treatment instrument insertion port, not shown.

In addition, the distal end portion 15 of the insertion portion 11 of the endoscope 2 is provided with the observation portion 28, the operation wire 126 for switching the focus of the observation portion 28, the first illumination window 29b, the second illumination window 30b, and the air/water feeding nozzle 34 linked to the air/water feeding conduit 106.

According to the above-described arrangement of these components, as shown in FIG. 5, a linear line La connecting the centers (point Pa1 and point Pa2 in FIG. 5) of the two illumination windows, that is, the first illumination window 29b and the second illumination window 30b located on both sides of the objective lens 118 of the observation optical system 115 intersects with a linear line Lb connecting the center (point Pb1 in FIG. 5) of the objective lens 118 and the center (point Pb2 in FIG. 5) of the operation wire 126.

That is, the observation portion 28 provided at the distal end portion 15 of the insertion portion 11 includes a mechanism for switching the focus of the observation portion 28 (advancing and retracting mechanism of the operation wire 126), which results in the shape in which one side portion of the observation portion 28 is protruded. If the distal end portion 15 is formed in accordance with the protruded shape, a dead space is likely to be created on both sides of the protruded part. In the embodiment of the present invention, in order to effectively use such dead spaces, the objective lens 118, the operation wire 126, and the two illumination windows, that is, the first illumination window 29b and the second illumination window 30b located on the both sides of the objective lens 118, are disposed as described above. As a result, the space of the distal end portion 15 of the insertion portion 11 is effectively used without creating a dead space, to thereby enable the reduction in the diameter of the distal end portion 15 of the insertion portion 11.

Furthermore, the distal end of the operation wire 126 is disposed at a position between the air/water feeding conduit 106 extended from the proximal end side of the insertion portion 11 to be linked to the proximal end portion of the air/water feeding nozzle 34 and the signal cable 125 extended from the proximal end side of the insertion portion 11 to be linked to the observation portion 28, and between the air/water feeding nozzle 34 and the observation portion 28. Specifically, as shown in FIG. 5, the distal end of the operation wire 126 is disposed between the tangential lines Ld1 and Ld2 of the outer diameter of the observation portion which are parallel to a linear line Lc connecting the center (point Pb1 in FIG. 5) of the objective lens 118 and the center (point Pc in FIG. 5) of the inlet of the air/water feeding nozzle 34. Therefore, such a configuration enables space-saving and reduced outer diameter of the distal end portion.

Thus, according to the embodiment of the present invention, the distal end portion 15 of the insertion portion 11 of the endoscope 2 is configured such that the first distal end surface 23a on which the objective lens 118 is provided is formed at the position protruded most in the distal end direction, then, the head top surface of the head top portion 34b of the air/water feeding nozzle 34 is formed at a position protruded in the distal end direction, and subsequently the third distal end surface 23c on which the first illumination window 29b and the second illumination window 30b are provided is formed at a position protruded in the distal end direction. Therefore, it is easy to bring the objective lens 118 on the first distal end surface 23 a protruded most in the distal end direction in good contact with the object to be observed. In addition, even if the objective lens 118 obliquely contacts the observation surface of the observation object (or contacts the observation surface so as to have a gradient), the head top surface of the head top portion 34b of the air/water feeding nozzle 34 contacts the observation object, to thereby surely prevent the third distal end surface 23c on which the first illumination window 29b and the second illumination window 30b are provided from contacting the observation object. Therefore, even if observation is performed for a long time with the observation window of the observation portion contacting the site to be observed in the body cavity or the like, the site to be observed is not affected by the heat, which enables the observation to be performed sufficiently and enables the observation performance to be improved.

The present application is filed and claims priority on the basis of Japanese Patent Application No. 2015-098450 filed in Japan on May 13, 2015, the disclosed contents of which are incorporated in the description, the claims and the drawings of the present application by reference.

## Claims

1. An endoscope comprising:
an insertion portion formed so as to extend in a longitudinal direction and configured to be inserted into a subject;
an observation window arranged at a distal end of the insertion portion, and configured to allow at least observation in a state of contacting an observation object; and
an illumination window configured to apply illumination light toward an observation field of view of the observation portion,
wherein the illumination window is arranged on a proximal end side in the longitudinal direction with respect to the observation window, and a distal end of a protruding portion is arranged between the observation window and the illumination window in the longitudinal direction.

2. The endoscope according to claim 1, wherein the distal end of the protruding portion is a head top portion of a nozzle configured to spout fluid toward the observation window.

3. The endoscope according to claim 2, wherein the observation window is arranged on a first distal end surface formed so as to be substantially orthogonal to the longitudinal direction, the nozzle is arranged on a second distal end surface adjacent to the first distal end surface, the second distal end surface being formed so as to be substantially orthogonal to the longitudinal direction on a more proximal end side than the first distal end surface, and the illumination window is arranged on a third distal end surface adjacent to the first distal end surface and the second distal end surface, the third distal end surface being formed so as to be substantially orthogonal to the longitudinal direction on a more proximal end side than the head top portion.

4. The endoscope according to claim 2, wherein a spouting port of the nozzle is open toward the observation window.

5. The endoscope according to claim 2, wherein the illumination system includes a plurality of illumination windows, switching of the observation portion between contact observation of the observation object and non-contact observation of the observation object is performed by operating an operation wire, and a linear line connecting centers of two illumination windows located on both sides of the observation window of the observation portion intersects with a linear line connecting a center of the observation window of the observation portion and a center of the operation wire.

6. The endoscope according to claim 5, wherein the operation wire is formed by a linear member, extended from a proximal end side of the insertion portion to be linked to the observation portion, and the operation wire is configured to switch focus of the observation portion by being advanced or retracted in the longitudinal direction of the insertion portion.

7. The endoscope according to claim 6, wherein a distal end of the linear member is disposed between the nozzle and the observation portion.

8. The endoscope according to claim 6, wherein a distal end of the linear member is disposed between an air/water feeding conduit extended from the proximal end side of the insertion portion and linked to a proximal end portion of the nozzle and a signal line extended from the proximal end side of the insertion portion and linked to the observation portion.

9. The endoscope according to claim 3, wherein adjacent parts of the first distal end surface, the second distal end surface, and the third distal end surface are formed by inclined planes to link the first distal end surface, the second distal end surface, and the third distal end surface with one another.
